# EUROPEAN PATENT SPECIFICATION

(11) **EP 1 880 219 B1**
(45) Date of publication and mention of the grant of the patent: **20.07.2011**
(21) Application number: 06724680.1
(22) Date of filing: 02.05.2006
(51) Int. Cl.: G01N 33/564, G01N 33/50, C07K 16/28

(54) **GPR18 AS A BIOMAKER FOR TH1 MEDIATED IMMUNE RESPONSE**
GPR18 ALS BIOMARKER FÜR TH1-VERMITTELTE IMMUNANTWORT
GPR18 UTILE COMME BIOMARQUEUR POUR LA REPONSE IMMUNE INDUITE PAR TH1

(30) Priority: 03.05.2005 GB 0508988
(43) Date of publication of application: 23.01.2008
(73) Proprietor: Novartis AG, 4056 Basel (CH)
(72) Inventor: WERNER, Gudrun, 1235 Vienna (AT); LAMETSCHWANDTNER, Günther, 1235 Vienna (AT); GUENTHER, Claudia, 1235 Vienna (AT); CARBALLIDO HERRERA, José, M., 1235 Vienna (AT)
(74) Representative: Schaller, Hans
(86) International application number: PCT/EP2006/004096
(87) International publication number: WO 2006/117194

(56) References cited:
- EP-B- 1 121 431
- WO-A-03/088808
- US-A1- 2004 018 522
- LAMETSCHWANDTNER GUNTHER ET AL: "Sustained T-bet expression confers polarized human TH2 cells with TH1-like cytokine production and migratory capacities" JOURNAL OF ALLERGY AND CLINICAL IMMUNOLOGY, vol. 113, no. 5, May 2004 (2004-05), pages 987-994, XP002400419 ISSN: 0091-6749
- WELL T N C ET AL: "CHEMOKINE RECEPTORS AND THEIR ANTAGONISTS IN ALLERGIC LUNG DISEASE" INFLAMMATION RESEARCH, BIRKHAEUSER VERLAG, BASEL, CH, vol. 48, no. 7, 1999, pages 353-362, XP000882070 ISSN: 1023-3830
- KENNEDY KEVIN J ET AL: "Role of chemokines in the regulation of Th1/Th2 and autoimmune encephalomyelitis" JOURNAL OF CLINICAL IMMUNOLOGY, vol. 19, no. 5, September 1999 (1999-09), pages 273-279, XP002400420 ISSN: 0271-9142

## Description

The present invention relates to a receptor, such as the use of the G protein-coupled receptor GPR18.

G protein-coupled receptors (GPRs) constitute a major class of proteins responsible for transducing a signal within a cell and represent one of the largest families of proteins with over 350 reported genes in the human genome.

GPRs have 3 structural domains: an amino terminal extracellular domain, a transmembrane domain containing 7 transmembrane segments, 3 extracellular loops and 3 intracellular loops and a carboxy terminal intracellular domain. Upon binding of a ligand to an extracellular portion of a GPR, a signal is transduced within the cell that results in a change in a biological or physiological property of the cell. GPRs, along with G-proteins and effectors such as e.g. intracellular enzymes and channels modulated by G-proteins, are the components of a modular signaling system that connects the state of intracellular second messengers to extracellular inputs.

GPRs therefore are a major target for drug action and development. Accordingly, it is valuable to the field of pharmaceutical development to identify and characterize previously unknown GPRs. As a novel GPR, the G protein-coupled receptor GPR18 was identified (see e.g. Gantz I. et al., Genomics 1997, 42:462-466). Little is known about the function of GPR18, e.g. beside missing evidence also its ligand is unknown.

US 20040018522 reports differences in peripheral blood cell gene expression as measured with DNA microarrays between healthy volunteers or non-autoimmune (ALS) patients and MS patients. GPR18 gene is listed in Table 4 as one gene (among many others) that is up-regulated in samples from MS patients.

WO 03088808 discloses the use of so-called TAT polypeptides, among those is disclosed GPR18 derived polypeptides, in particular for the treatment of cancer. The association of GPR18 gene expression and cancer derives from gene expression profiles in normal tissue and tumors.

EP1121431 discloses human G protein-coupled receptors (GPCRs) which have been altered such that altered GPCRs become constitutively expressed. Also disclosed is the use of the altered GPCRs for screening therapeutic compounds. GPR18 is mentioned in the examples.

Surprisingly we have now found a strong correlation of GPR18 expression with the Th1 phenotype of human T cells and in consequence a strong correlation of GPR18 with Th1 driven autoimmune disorders. We have also found that GPR18 is much lower expressed in Th-2-cells than in Th1 cells. GPR18 may thus be used as a target for the specific inhibition of Th1 mediated, e.g. Th1 associated, immune responses, e.g. as a biomarker for specifically monitoring the infiltration of Th1 cells in inflamed skin.

E.g. psoriasis is an inflammatory skin disease mediated by exacerbated Th1 responses. Thus, specific inhibition of Th1 cell activation and cytokine production, will result in amelioriation of the psoriatic phenotype. However, a general inhibition of T cell responses would render a patient vulnerable against all kinds of infectious agents. Therefore, an effective and save drug against Th1 mediated diseases, should rather specifically inhibit enhanced Th1 responses.

The dominant role of T-bet inducing Th1 cell differentiation makes this molecule and its downstream pathway targets excellent candidates for therapeutic intervention in psoriasis and autoimmune diseases. To this end, T-bet, the key transcription factor for Th1 cell function, (see e.g. Szabo et al., Cell. 2000 Mar 17;100(6):655-69) is ectopically expressed in non T-bet expressing skin derived Th2 cells. The transcriptional changes, which are directly or indirectly (via increased responsiveness to IL-12) induced by T-bet are followed by comparing the mRNA levels of genes by microarray analysis (U133A and B Affymetrix microarrays) of T-bet transfected Th2 cells with control transfected cells (Lametschwandtner et al., J Allergy Clin. Immunol. 2004,113(5):987-94).

Surprisingly we have found that the gene which encodes the G-protein coupled receptor GPR18 is induced by T-bet and TCR stimulation.

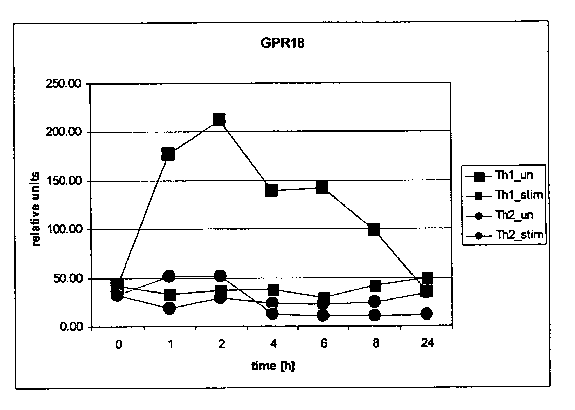

The specific induction of GPR18 in Th1 polarized skin derived T cells is verified by real time PCR. Results are set out in Figure 1 and in TABLE 1 below. Furthermore, GPR18 could be specifically induced by Th1 polarizing conditions in T helper cells generated from naïve precursors.

Thus, we have foung a strong correlation of GPR18 expression with the Th1 phenotype of human T cells and the Th1 driven autoimmune disease psoriasis. GPR18 has thus found to be a biomarker for specifically monitoring the infiltration of Th1 cells in inflamed skin and generally as a target for the specific inhibition of Th1 mediated immune responses.

In several aspects the present application discloses.
1. GPR18 for use, e.g., or the use of GPR18
   1.1 as a target for, e.g. specific, inhibition of Th1 mediated immune responses;
   1.2 as a target for, e.g. specific, inhibition of Th1 cell activation;
   1.3 for diagnosing disorders mediated, e.g. associated with, e.g. driven, by, e.g. exacerbated, Th1 cell activation;
   1.4 for diagnosing immune disorders mediated, e.g. associated with, e.g. driven by, e.g. exacerbated, Th1 cell activation;
   1.5 for diagnosing autoimmune disorders mediated, e.g. associated with, e.g. driven by, e.g. exacerbated, Th1 cell activation;
   1.6 for diagnosing inflammatory disorders associated with immune, such as autoimmune, disorders, mediated, e.g. associated with, e.g. driven by, e.g. exacerbated, Th1 cell activation;
   1.7 for diagnosing psoriasis, mediated, e.g. associated with, e.g. driven by, e.g. exacerbated, Th1 cell activation;
   1.8 for monitoring the infiltration of Th1 cells in inflamed skin.

In several other aspects the present application discloses
2. GPR18, e.g. or the use of GPR18, for any one of the uses as indicated under 1.1 to 1.8, 2.1 as a biomarker,
   e.g. in a sample of an individual,
   e.g. in a sample of a body fluid or a tissue sample of an individual,
   e.g. in a biopsy-sample of an individual,
   e.g. skin biopsy-sample, of an individual.

GPR18 as indicated under any of 1. or 2, above includes GPR18 in any form, e.g. in the form of
- a nucleic acid encoding GPR18, e.g. including a nucleic acid encoding a derivative of GPR18,
- GPR18 protein, e.g. including protein which is a GPR18 derivative, or
- GPR18 secreting cells, e.g. or a derivative of GPR18 secreting cells.

"A derivative" of GPR18 nucleic acid or protein, e.g. in secreting cells, according to the present invention includes a fragment, a mutant, a variant, an homolog or a modification of a GPR18 protein, or of a nucleic acid encoding GPR18, which retains, e.g. essentially, the biological function of GPR18, e.g. which retains, e.g. essentially, the biological function of GPR18 as a mediator in Th1 mediated immune responses. GPR18-secreting cells, e.g. including GPR18 producing cells, include antigen presenting cells (APC), such as e.g. monocytes and dendritic cells (DC).

Thus, GPR18 for use as disclosed in the present application icludes splice variants encoded by mRNA generated by alternative splicing of a primary transcript, amino acid mutants, posttranslational modifications, such as glycosylation and phosphorylation variants, and modifications which are covalent derivatives of GPR18 and which retain the biological function of GPR18. Exemplary GPR18 derivatives include modifications wherein the GPR18 protein is covalently modified by substitution, e.g. substitution originating from appropriate means, e.g. chemical or enzymatic means, by a moiety in the GPR18 protein. Such a moiety e.g. includes one or more amino acids, e.g. naturally occurring amino acids and other than naturally occurring amino acids, and/or a detectable moiety. A detectable moiety includes an enzyme, a radioisotope, tags, toxins and genes such as oncogenes and tumour suppressor genes. GPR18 derivatives further includ naturally occurring variants of GPR18, e.g. provided within a particular species. Such a variant may be encoded by a related gene of the same gene family, by an allelic variant of a particular gene, or represent an alternative splicing variant of the GPR18 gene.

A GPR18 derivative as disclosed herein also includes fragments of a nucleic acid encoding GPR18, or of the GPR18 protein, and comprises individual GPR18 domains and smaller polypeptides derived from GPR18 domains. Preferably, smaller polypeptides derived from GPR18 according to the invention define a single functional activity which is characteristic of GPR18. Fragments may in theory be of almost any size, as long as they retain the biological characteristic of GPR18. Preferably, fragments will be between 12 and 210 nucleic acids in length or between 4 and 70 amino acids, respectively. Longer fragments are regarded as truncations of the full-length GPR18.

Derivatives of GPR18 as disclosed herein also comprise mutants thereof, which may contain amino acid deletions, additions or substitutions, subject to the requirement to retain the biological function of GPR18. Conservative amino acid substitutions may be made substantially without altering the nature of GPR18, e.g. by truncations from the 5' or 3' ends. Deletions and substitutions also include deletions and substitutions in fragments of GPR18. GPR18 mutants may be produced from a DNA encoding GPR18 which has been subjected to *in vitro* mutagenesis resulting e.g. in an addition, exchange and/or deletion of one or more amino acids in GPR18. For example, substitutional, deletional or insertional variants of GPR18 can be prepared by recombinant methods and screened for functional similarity to the native forms of GPR18.

Derivatives of GPR18 as disclosed herein also include GPR18 homologs, preferably GPR18 homologs retain substantial homology with GPR18. As used herein, "homology' means that GPR18 and a GPR18 homolog share sufficient characteristics to retain the biological function of GPR18. Preferably, homology is used to refer to sequence identity. Thus, the derivatives of GPR18 preferably retain substantial sequence identity with the nucleic acid sequence as given in Gantz I. et al., Genomics 42, 462-466, 1997 or the translated protein sequence thereof.

"Substantial homology", where homology indicates sequence identity, means more than 50% sequence identity, preferably more than 75% sequence identity and even more preferably a sequence identity of 80% and more, e.g. 90% and more, such as 90%, 91%, 92%, 93%, 94%, 95%. 96%, 97%, 98% or 99%..

Preferably GPR18 is originating from a mammal.
The nucleic acid encoding GPR18 preferably has the nucleic acid sequence as described in Gantz I. et al., Genomics 42, 462-466, 1997 and GenBank Accession No. L42324.
The GPR18 protein preferably corresponds to the translated protein sequence of the above mentioned nucleic acid.

Biomarker as disclosed herein means that determination (= detection and/or quantification) of a GPR18 molecule in a sample of an individual is an indicator for a disorder or disease as such and/or is useful for monitoring the status of a disorder or disease related with Th1 mediated immune responses, e.g. autoimmune responses, e.g. immune response related with inflammation, such as inflammatory skin diseaseas, e.g. including psoriasis.

In another aspect the present application discloses a method for diagnosing a disorder or disease which is mediated, e.g. associated with, e.g. driven by, e.g. exacerbated, Th1 cell activation, comprising
a) providing a sample of an individual,
b) determining the level of GPR18 in said sample,
c) comparing the level of GPR18 as determined in step b) with a reference level from a sample of a healthy control individual, and
d) diagnosing a disorder or disease which is mediated, e.g. associated with, e.g. driven by, e.g. exacerbated, Th1 cell activation by determination whether the level of GPR18 as determined in step b) is, e.g. significantly, different from said reference level.

In another aspect the present application discloses a method for monitoring the therapeutic efficacy in the treatment of an individual with a substance which is expected to have an effect on reducing or curing a disorder or disease which is mediated, e.g. associated with, e.g. driven by, e.g. exacerbated, Th1 cell activation, which method comprises determining the level of GPR18 in a sample of said individual and comparing that level with the level of GPR18 prior to administration of said substance.

A sample of an individual according to a use or a method of the present application includes a sample of a body fluid or a tissue sample. A body fluid may be derived e.g. from blood, e.g. including isolated mononuclear cells, or from a blood fraction, e.g. including plasma or serum, preferably serum. A tissue sample may be a biopsy, e.g. such as a skin biopsy.

In another aspect the present application discloses the a use or a method of the present invention wherein a sample is a body fluid or a tissue sample of an individual, e.g. a body fluid may be derived from blood, e.g. isolated mononuclear cells, or from a blood fraction, e.g. plasma or serum, e.g. serum; e.g. the tissue sample may be a biopsy, e.g. such as a skin biopsy.

Detection means for determining the level of GPR18 include means as conventional, e.g. immunoassays, such as an immunodiagnostic method, an enzyme linked immunoassay (ELISAs); a fluorescence based assay, such as dissociation enhanced lanthanide fluoroimmunoassay (DELFIA), an radiometric assay or by carrying out a GPR18 specific Polymerase Chain Reaction (PCR); specifically detection means include a molecule which specifically recognizes GPR18, e.g. a molecule which is directly or indirectly detectable, preferably comprising an antibody, including antibody derivatives or fragments thereof, e.g. an antibody which recognizes GPR18, e.g. a label bearing GPR18 recognizing antibody. Such label may be a conventional label, e.g. biotin or an enzyme such as alkaline phosphatase (AP), horse radish peroxidase (HRP) or peroxidase (POD) or a fluorescent molecule, e.g. a fluorescent dye, such as e.g. fluorescein isothiocyanate. Preferably the label is biotin. The label bearing molecule, e.g. the label bearing antibody, may be detected according to methods as conventional, e.g. via fluorescence measurement or enzyme detection methods.

An antibody fragment or antibody derivative includes a fragment or a derivative, e.g. chemically or enzymatically modified, of an antibody which still is capable of recognising GPR18.

GPR18-secreting cells in a sample of a body fluid of an individual, e.g. blood, may be determined by a method as conventional, e.g. by the following method:
Cells may be purified, e.g. separated by a density gradient, from the sample, e.g. blood, and the purified cells obtained are stained. Anti-GPR18 antibodies, e.g. fluorescence labeled anti-GPR18 antibodies, are added to the stained cell preparation, optionally after stimulation of the cells, e.g. with interleukin-4, and the level of GPR18-secreting cells is determined.

Optionally, GPR18 comprised in the sample or the GPR18 recognizing, e.g. detectable, molecule comprised in the detection means is immobilized on a solid phase. An appropriate solid phase includes e.g. conventional solid phases used for immobilization, e.g. a plastic plate like a polystyrene or polyvinyl plate, especially a microtiter plate. Also microbeads can be used as a solid phase, e.g. coated microbeads. The solid phase can be coated with a coating material the nature of which depends e.g. on the label comprised in the detection means. The coating material should be able to bind to the label, e.g. if the label is biotin a coating material includes streptavidin, e.g. covalently bound to the solid phase.
Preferably determination of GPR18 is carried out by using a molecule which specifically recognizes the GPR18, e.g. an antibody, an antibody derivative, or an antibody fragment, such as an anti GPR18 antibody, e.g. a commercially available GPR18 specific antibody. Detection of GPR18-antibody formation preferably is carried by an immunodiagnostic assay method.

In another aspect the present application discloses a method for diagnosing a disorder or disease which is mediated, e.g. associated with, e.g. driven by, e.g. exacerbated, Th1 cell activation, according to the present invention wherein the level of GPR18 is determined by use of an GPR18 specific antibody.

Disorders and diseases mediated, e.g. associated with, e.g. driven by, e.g. exacerbated Th1 cell activation for which GRPR18 may be used according to the present invention, e.g. for which GPR18 may be used as set out under 1.1 to 1.8 above, include disorders and diseases of the immune system, e.g. autoimmune disorders and diseases, e.g. disorders and diseases associated with inflammation, which are mediated by Th1 cell activation, such as inflammatory skin diseases, psoriasis, rheumatoid arthritis, inflammatory bowel disease, e.g. Crohn's disease, multiple sclerosis, diabetes, lupus, e.g. systemic lupus erythematosus, disorders associated with transplantation, e.g. including transplant rejection crisis and other disorders following transplantation, such as organ or tissue transplant rejection, e.g. for the treatment of recipients of e.g. heart, lung, combined heart-lung, liver, kidney, pancreatic, skin, corneal transplants, graft versus host disease, such as following bone marrow transplantation; and cancer and diseases associated therewith, e.g. cancer associated with Th1 cell activation.

Disorders and diseases for which GRPR18 may be used according to the present invention include specifically psoriasis and conditions which arise in a patient having psoriasis, e.g. psoriatic arthritis.

Psoriasis is an autoimmune disorder which is related with Th1 cell activation and which is associated with inflammatory skin disease in which skin cells replicate at an extremely rapid rate. New skin cells are produced about 8 times faster than normal - over several days instead of a month - but the rate at which old cells slough off remains unchanged. This causes cells to build up on the skin's surface, forming thick patches, or plaques, of red scores (lesions) covered by flaky, silvery-white dead skin cells (scales).

Rarely life-threatening, at its mildest, psoriasis can be itchy and sore. At its worst, it's painful, disfiguring and debilitating. About 2/3s of the people with psoriasis have a mild form of the disease. About 1/3 have moderate or severe psoriasis. Psoriasis can affect people at any age, but it most often strikes those between the ages of 15 and 35.

There are 5 forms of psoriasis. Plaque psoriasis is the most common - affecting 4 out of 5 people who have psoriasis. Plaque psoriasis may start with small reed bumps and progress to larger lesions.

The plaques of psoriasis occur most frequently on the elbows, knees, other parts of the legs, scalp, back, face, palms and sole of the feet. Psoriasis can also affect the fingernails and toenails, causing pitting, discoloration or tissue buildup around the nails.

According to the National Institute of Arthritis and Musculoskeletal and Skin Diseases, about 15% of people with psoriasis also get psoriatic arthritis, which can be progressively disabling if untreated.

It is believed that T lymphocytes (T cells) play an important role in psoriasis and it was found that Th1 cell activation plays a major role. Psoriasis also has a genetic component: in about 1/3 of psoriasis cases, there is a family history of the disease.

T cells circulate throughout the body, orchestrating the immune system's response to foreign invaders like bacteria or viruses. In people with psoriasis, the defective T cells are overactive and migrate to the skin as if to heal a wound or ward of an infection. This process leads to the rapid growth of skin cells, triggering inflammation and development of lesions. Until now, no single test exists to diagnose psoriasis, but a dermatologist can usually determine it by appearance of the skin and by locking at an individual's personal and family medical history.

A dermatologist can usually determine psoriasis by appearance of the skin and by locking at an individual's personal and family medical history, but, until now, no single test exists to diagnose psoriasis.

In another aspect the present application discloses a kit for screening for an agent for the treatment of a disorder or disease which is mediated, e.g. associated with, e.g. driven by, e.g. exacerbated, Th1 cell activation, e.g. psoriasis, in a sample of an individual comprising
a) a molecule which recognizes GPR18, optionally in a labeled form,
b) instructions for use,
c) optionally detection means, and
d) optionally a solid phase.

In another aspect the present application discloses a kit or diagnosing of a disorder or disease which is mediated, e.g. associated with, e.g. driven by, e.g. exacerbated, by Th1 cell activation in a sample of an individual comprising
a) a molecule which recognizes GPR18, optionally in a labeled form,
b) instructions for use,
c) optionally detection means,
d) optionally a solid phase.

Such kit may further comprise a substantial component, e.g. including an appropriate environment of a sample to be tested and, e.g. appropriate means to determine GPR18 in a sample to be tested.

In a further aspect the present invention provides an assay for identifying an agent that modulates Th1 cell activation comprising
a) determining the level of GPR18 in Th1 cells, in the absence and in the presence of a candidate compound which is expected to modulate the level of GPR18,
b) identifying a candidate compound which modulates the level of GPR18 as determined in step a) as an agent, e.g. and
   using such agent as a pharmaceutical in the treatment of disorders or diseases mediated, e.g. associated with, e.g. driven by, e.g. exacerbated, Th1 cell activation.

A GPR18 specific test system may comprise GPR18 secreting cells or it may be a system having a specific GPR18 activity.

The level of GPR18 is determined as appropriate, e.g. as described herein.

A candidate compound as described herein is a compound which may be expected to modulate the level of Th1 cell activity via modulating the level of GPR18, e.g. GPR18 activity or GPR18 secreting cells, and includes compound(s)(libraries) from which its influence on GPR18 can be determined. Compound (libraries) include for example oligopeptides, polypeptides, proteins, antibodies, mimetics, small molecules, e.g. low molecular weight compounds (LMWs).

An agent is a candidate compound which modulates, e.g. decreases the level of Th1 activation via GPR18 inhibition, e.g. in a sample form a patient, e.g. a blood sample, such as serum, or a skin biopsie. An agent includes oligopeptides, polypeptides, proteins, antibodies, mimetics, small molecules, e.g. low molecular weight compounds (LMW's).

In another aspect the present invention provides an agent identified by an assay or a method of the present invention.

An agent of the present invention may exhibit pharmacological activity and is therefore useful as a pharmaceutical. An agent of the present invention may show therapeutic activity, e.g. in disorders or diseases mediated, e.g. associated with, e.g. driven by, e.g. exacerbated, Th1 cell activation.

In another aspect the present invention provides a GPR18 inhibitor of the present invention for use in the treatment of disorders mediated, e.g. associated with, e.g. driven by, e.g. exacerbated by, Th1 cell activation, wherein said GPR18 inhibitor is an antibody which decreases the level of Th1 cell activation via GPR18 inhibition.

For pharmaceutical use an agent of the present invention for treatment includes one or more, preferably one, antibody of the present invention, e.g. a combination of two or more antibodies of the present invention.

In another aspect the present invention provides the use of an antibody of the present invention for the manufacture of a medicament for the treatment of disorders or diseases mediated, e.g. associated with, e.g. driven by, e.g. exacerbated by, Th1 cell activation.

In another aspect the present invention provides a pharmaceutical composition comprising the GPR18 inhibitor of the present invention beside at least one pharmaceutical excipient, e.g. appropriate carrier and/or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers, for use in the treatment of disorders mediated by Th1 cell activation, wherein said GPR18 inhibitor is an antibody which decreases the level of Th1 cell activation via GPR18 inhibition.

The treatment of disorders or diseases mediated, e.g. associated with, e.g. driven by, e.g. exacerbated by, Th1 cell activation may be accomplished by administering an effective amount of an antibody of the present invention to a subject in need of such treatment.

For such treatment, the appropriate dosage will, of course, vary depending upon, for example, the chemical nature and the pharmakokinetic data of a compound of the present invention used, the individual host, the mode of administration and the nature and severity of the conditions being treated. However, in general, for satisfactory results in larger mammals, for example humans, an indicated daily dosage includes a range
- from about 0.001 g to about 1.5 g, such as 0.001 g to 1.5 g;
- from about 0.01 mg/kg body weight to about 20 mg/kg body weight, such as 0.01 mg/kg body weight to 20 mg/kg body weight,
for example administered in divided doses up to four times a day.

An antibody of the present invention may be administered by any conventional route, for example enterally, e.g. including nasal, buccal, rectal, oral, administration; parenterally, e.g. including intravenous, intramuscular, subcutanous administration; or topically; e.g. including epicutaneous, intranasal, intratracheal administration; via medical devices for local delivery, e.g. stents,
e.g. in form of coated or uncoated tablets, capsules, (injectable) solutions, solid solutions, suspensions, dispersions, solid dispersions; e.g. in the form of ampoules, vials, in the form of creams, gels, pastes, inhaler powder, foams, tinctures, lip sticks, drops, sprays, or in the form of suppositories.

For topical use, e.g. including administration to the eye, satisfactory results may be obtained with local administration of a 0.5-10 %, such as 1-3% concentration of active substance several times daily, e.g. 2 to 5 times daily.

An antibody of the present invention may be administered in the form of a pharmaceutically acceptable salt, e.g. an acid addition salt or metal salt; or in free form; optionally in the form of a solvate. An agent of the present invention in the form of a salt may exhibit the same order of activity as an agent of the present invention in free form; optionally in the form of a solvate.

An antibody of the present invention may be used for pharmaceutical treatment according to the present invention alone, or in combination with one or more other pharmaceutically active agents.

Combinations include fixed combinations, in which two or more pharmaceutically active agents are in the same formulation; kits, in which two or more pharmaceutically active agents in separate formulations are sold in the same package, e.g. with instruction for coadministration; and free combinations in which the pharmaceutically active agents are packaged separately, but instruction for simultaneous or sequential administration are given.

### Description of the Figures

### Figure 1

In Figure 1 is shown the expression of GRP18 ion Th1 unstimulated, Th1 stimulated, Th2 unstimulated and Th2 stimulated cells.

### EXAMPLE 1

### Real time PCR

CD4 Tcells are either left unstimulated or stimulated as described (Lametschwandtner et al., J Allergy Clin Immunol 2004: 113 (5), p987-994). Total cellular RNA is prepared using the Qiagen RNeasy Mini-kit. Quantitative Real-time PCR is performed on a Gene Amp 9600 Thermocycler (Perkin Elmer) by using gene-specific primers and probes, which are designed with PrimerExpress Software (Applied Biosystems). Amplification is performed using the following settings: 2 minutes 50°C, 30 minutes at 60°C and 5 minutes at 95°C, followed by 40 cycles of 20 seconds at 94°C and 1 minute at 62°C. Levels of GPR18 RNA are calculated using the deltaCt method (Sequence detector 3.1, Applied biosystems) and expressed as percent of the housekeeping gene HPRT.

Results are set out in TABLE 1 below and in Figure 1.

**TABLE 1**

| GPR18 | Th1_un | Th1_stim | Th2_un | Th2_stim |
|---|---|---|---|---|
| 0 | 41.90 | 41.90 | 32.36 | 32.36 |
| 1 | 33.45 | 177.15 | 18.56 | 51.23 |
| 2 | 36.86 | 212.14 | 28.72 | 51.94 |
| 4 | 37.50 | 139.47 | 23.73 | 12.63 |
| 6 | 29.32 | 141.91 | 22.53 | 10.29 |
| 8 | 42.04 | 97.94 | 24.32 | 10.33 |
| 24 | 50.00 | 34.99 | 34.03 | 12.07 |

From TABLE 1 it is immediately evident that GPR18 in unstimulated TH1 cells and in unstimulated and stimulated Th2 cells is practically not expressed whereas GPR18 is highly expressed in stimulated Th1 cells. These results are also set out in Figure 1.

## Claims

1. GPR18 inhibitors for use in the treatment of disorders mediated by Th1 cell activation, wherein said GPR18 inhibitor is an antibody which decreases the level of Th1 cell activation via GPR18 inhibition.

2. The GPR18 inhibitors of Claim 1 for the use according to claim1 wherein said disorders mediated by Th1 cell activation are selected among inflammatory skin diseases, psoriasis, rheumatoid arthritis, inflammatory bowel disease, multiple sclerosis, diabetes, lupus, disorders associated with transplantation, cancer associated with Th1 cell activation.

3. The GPR18 inhibitors of Claim 1 or 2 for the use according to claims 1 and 2, wherein said disorder mediated by Th1 cell activation is psoriasis.

4. A pharmaceutical composition comprising a GPR18 inhibitor according to any one of claims 1 to 3 together with at least one pharmaceutical excipient for use in the treatment of disorders mediated by Th1 cell activation, wherein said GPR18 inhibitor is an antibody which decreases the level of Th1 cell activation via GPR18 inhibition.

5. An assay for identifying an agent that modulates Th1 cell activation comprising
a) determining the level of GPR18 in Th1 cells, in the absence and in the presence of a candidate compound which is expected to modulate the level of GPR18,
b) identifying a candidate compound which modulates the level of GPR18 as determined in step a) as an agent.

## Patentansprüche

1. GPR18-Inhibitoren zur Verwendung bei der Behandlung von durch Th1-Zellaktivierung vermittelten Erkrankungen, wobei es sich bei dem GPR18-Inhibitor um einen Antikörper handelt, der über die GPR18-Hemmung den Grad der Th1-Zellaktivierung herabsetzt.

2. GPR18-Inhibitoren nach Anspruch 1 zur Verwendung gemäß Anspruch 1, wobei die durch Th1-Zellaktivierung vermittelten Erkrankungen unter entzündlichen Hautkrankheiten, Psoriasis, rheumatoider Arthritis, entzündlicher Darmkrankheit, multipler Sklerose, Diabetes, Lupus, Erkrankungen im Zusammenhang mit Transplantation, Krebs im Zusammenhang mit Th1-Zellaktivierung ausgewählt sind.

3. GPR18-Inhibitoren nach Anspruch 1 oder 2 zur Verwendung gemäß Anspruch 1 und 2, wobei es sich bei der durch Th1-Zellaktivierung vermittelten Erkrankung um Psoriasis handelt.

4. Pharmazeutische Zusammensetzung, umfassend einen GPR18-Inhibitor gemäß einem der Ansprüche 1 bis 3 zusammen mit wenigstens einem pharmazeutischen Hilfsstoff, zur Verwendung bei der Behandlung von durch Th1-Zellaktivierung vermittelten Erkrankungen, wobei es sich bei dem GPR18-Inhibitor um einen Antikörper handelt, der über die GPR18-Hemmung den Grad der Th1-Zellaktivierung herabsetzt.

5. Testverfahren zur Identifizierung eines Agens, das die Th1-Zellaktivierung moduliert, umfassend:
a) Bestimmen des GPR18-Spiegels in Th1-Zellen in Abwesenheit bzw. in Gegenwart einer Kandidatenverbindung, von der erwartet wird, dass sie den GPR18-Spiegel moduliert;
b) Identifizieren einer Kandidatenverbindung, die den GPR18-Spiegel moduliert, wie in Schritt a) bestimmt, als Agens.

## Revendications

1. Inhibiteurs de GPR18 pour utilisation dans le traitement de troubles médiés par l'activation de cellules Th1, où ledit inhibiteur de GPR18 est un anticorps qui diminue le taux d'activation de cellules Th1 via l'inhibition de GPR18.

2. Inhibiteurs de GPR18 de la revendication 1 pour l'utilisation selon la revendication 1, où lesdits troubles médiés par l'activation de cellules Th1 sont choisis parmi des maladies inflammatoires de la peau, le psoriasis, la polyarthrite rhumatoïde, le syndrome abdominal inflammatoire, la sclérose en plaques, le diabète, le lupus, des troubles associés à une transplantation, un cancer associé à l'activation de cellules Th1.

3. Inhibiteurs de GPR18 de la revendication 1 ou 2 pour l'utilisation selon les revendications 1 et 2, où ledit trouble médié par l'activation de cellules Th1 est le psoriasis.

4. Composition pharmaceutique comprenant un inhibiteur de GPR18 selon l'une quelconque des revendications 1 à 3 conjointement avec au moins un excipient pharmaceutique pour utilisation dans le traitement de troubles médiés par l'activation de cellules Th1, où ledit inhibiteur de GPR18 est un anticorps qui diminue le taux d'activation de cellules Th1 via l'inhibition de GPR18.

5. Essai pour identifier un agent qui module l'activation de cellules Th1 comprenant
a) la détermination du taux de GPR18 dans des cellules Th1, en l'absence et en présence d'un composé candidat pour lequel il est attendu qu'il module le taux de GPR18,
b) l'identification d'un composé candidat qui module le taux de GPR18 tel que déterminé dans l'étape a) en tant qu'agent.
